# EUROPEAN PATENT APPLICATION

(11) **EP 1 674 036 A1**
(43) Date of publication of application: **28.06.2006**
(21) Application number: 04787930.9
(22) Date of filing: 21.09.2004
(51) Int. Cl.: A61B 5/04

(54) **CLOTHES FOR BABIES WITH BIOMETRIC SENSOR, SHEET FOR BABIES WITH BIOMETRIC SENSOR AND BIOMETRIC METHOD**

(30) Priority: 03.10.2003 JP 2003346300
(71) Applicant: Aprica Ikujikenkyukai Aprica Kassai Kabushikikaisha, Osaka-shi, Osaka 542-0082 (JP)
(72) Inventor: KASSAI, Kenzou, Chuo-ku, Osaka-shi, Osaka 5420083 (JP); SUZUKI, Sachiyo, APRICA KASSAI KABUSHIKI KAISHA, Chuo-ku, Osaka-shi, Osaka 5420082 (JP)
(74) Representative: Hofer, Dorothea
(86) International application number: PCT/JP2004/013747
(87) International publication number: WO 2005/032368

(57) **Abstract**

A living body sensor (30) using a conductive fiber as an electrode is incorporated into a band portion (2) of a clothing for infant (1), a cloth (7) between a conductive fabric (31) and a skin (6) is capacitance-coupled as a capacitance, a living body electric signal is extracted from the conductive fabric (31), and an electrocardiograhic waveform is outputted based on an output of the living body measuring sensor (30) by means of an impedance converter having a high input impedance and a low output impedance.

## Description

### TECHNICAL FIELD

The present invention relates to a clothing for infant provided with a living body measuring sensor, a sheet for infant provided with a living body measuring sensor and a living body measuring method, more particularly to a clothing for infant provided with a living body measuring sensor, an example of which is a clothing for infant such as a diaper cover in which a sensor for measuring an electrocardiogrphic waveform is incorporated, a sheet for infant provided with a living body measuring sensor such as a sheet of a baby carriage in which the sensor is incorporated and a living body measuring method.

### BACKGROUND ART

It is said that a number of infants and neonates die of a cardiac disease, respiratory disturbances, and sudden infant death syndrome in our country. In order to investigate a cause of the sudden infant death syndrome and make the diagnosis of / provide a medical treatment for the cardiac disease / respiratory disturbances of the infant and neotate, it is necessary to continuously measure an electrocardiogram by an electrocardiograph. Low birthweight babies (premature babies), the number of whom has tended to increase in recent years, also need to be monitored using continuously measured electrocardiograms in order to assure the growth after birth.

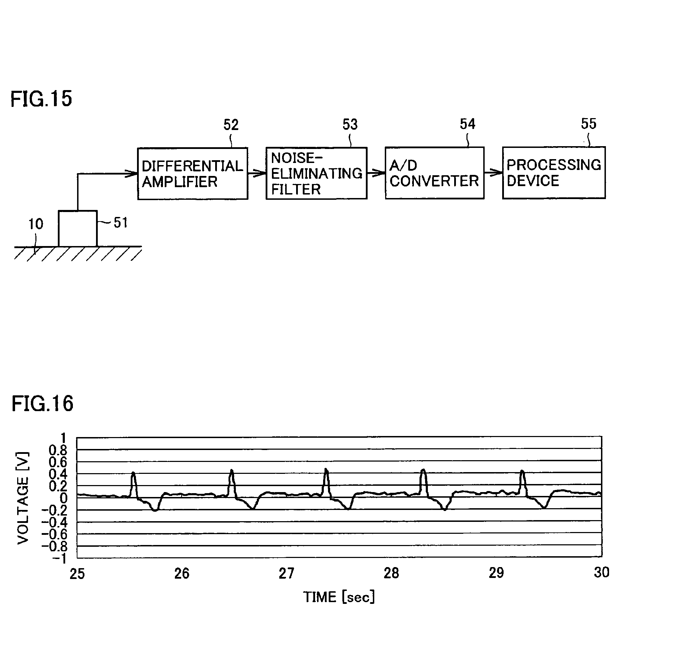

Fig. 15 is a schematic diagram of a conventional electrocardiograph. For the purpose of the measurement of the electrocardiogram, a fixed electrode 51 such as a silver / silver chloride electrode shown in Fig. 15 is made to closely contact a skin 10 as a body surface using a conductive paste in around a wrist or an ankle of a measuring subject, adsorbed to the skin 10 by reducing a pressure or pressurized by a belt to be thereby fixed thereto. A living body electric signal obtained from the fixed electrode 51 is amplified by a differential amplifier 52, and a noise component thereof is eliminated by a noise eliminating filter 53, and further, the living body electric signal is sampled by an A/D converter 54 to be thereby converted into a digital signal. The digital signal is then processed by a processing device 55 so that an electrocardiogram shown in Fig. 16 is recorded on a recorder or displayed in a waveform on a display screen.

However, the fixed electrode 51 directly contacts the skin 10 via the applied conductive paste so as to extract the electrocardiogram in the conventional device shown in Fig. 15. Therefore, there is a large burden on the skin in the case of the neonate and infant, which frequently accidentally separates the skin from the body surface as well as the electrode when it is removed. Further, an allergic reaction is induced in some cases because the metal electrode is used.

Japanese Unexamined Patent Application No. 2002-159458 recites a living body electric signal induction sensor and a recording system in which a conductive fiber is woven into a shoulder portion of a clothing, the living body electric signal is detected by the conductive fiber constituting an induction electrode, and the electrocardiogram is recorded on a recorder housed in a pocket of the clothing.

However, in the case of using the conductive fiber as the induction electrode, the conductive fiber may fail to closely contact the skin, which cannot assure an accurate electrocardiogram. Further, the conductive fiber includes the risk of inducing the allergic reaction in the same manner as in the metal fixed electrode.

### DISCLOSURE OF THE INVENTION

Therefore, an object of the present invention is to provide a clothing for infant provided with a living body measuring sensor, a sheet for infant provided with a living body measuring sensor and a living body measuring method capable of reducing a burden and invasiveness generated from a measurement on an infant.

The present invention relates to a clothing for infant provided with a living body measuring sensor for detecting a living body electric signal from a body surface of an infant, which comprises a conductive electrode incorporated into the clothing for infant and capacitance-coupled via an insulating member on the body surface of the infant.

According to the present invention, the conductive electrode is mounted on the body surface of the infant via the insulating member so that the burden generated from the measurement on the infant can be reduced and an electrocardiogram can be measured in a less invasive manner.

The conductive electrode is preferably a metal electrode.

The conductive electrode is preferably a conductive fiber.

The insulating member is preferably a thin cloth.

The clothing for infant is preferably a diaper cover, and the conductive electrode and the insulating member are incorporated into a band portion of the diaper cover.

The clothing for infant is preferably an upper wear, and the conductive electrode and the insulating member are incorporated into a band portion provided in a lower part of the upper wear.

The clothing for infant is preferably an upper wear, and the conductive electrode and the insulating member are incorporated into a breast portion of the upper wear.

The clothing for infant is preferably an upper wear, and the conductive electrode and the insulating member are incorporated into a breast portion and a back portion of the upper wear.

The clothing for infant is preferably a pressure sensitive adhesive sheet into which the conductive electrode is incorporated.

The present invention relates to a sheet for infant provided with a living body measuring sensor for detecting a living body electric signal from a body surface of an infant, which comprises a conductive electrode incorporated into the sheet for infant and capacitance-coupled via an insulating member on the body surface of the infant.

According to the present invention, the conductive electrode can be mounted on the body surface of the infant via the insulating member, for example, in the state in which the infant is sat or laid down on the sheet for infant. Accordingly, the infant can be monitored by continuously measuring the electrocardiogram in order to, for example, assure the growth after birth.

The sheet for infant is preferably a sheet with which the body surface makes a close contact when the infant is sat down.

The sheet for infant is preferably a sheet with which the body surface makes a close contact when the infant is laid down.

The present invention relates to a living body measuring method for extracting a living body electric signal from a body surface of an infant via a conductive electrode, wherein an insulating member is interposed between the body surface of the infant and the conductive electrode to enable capacitance coupling, and the living body electric signal is thereby outputted with a low impedance.

The insulating member is preferably a clothing worn by the infant.

The conductive electrode is preferably incorporated into the clothing worn by the infant and outputs the living body electric signal.

### BRIEF DESCRIPTION OF DRAWINGS

Fig. 1 shows a clothing for infant provided with a living body measuring sensor according to an embodiment of the present invention.
Fig. 2 shows the living body measuring sensor incorporated into the clothing for infant of Fig. 1.
Fig. 3 is a graph showing a relationship between a thickness of a cloth and a capacitance.
Fig. 4 is a graph showing a relationship between a frequency and an impedance.
Fig. 5 is a block diagram of a living body measuring device according to an embodiment of the present invention.
Fig. 6 shows an electrocardiographic waveform outputted from a processing device of the living body measuring device shown in Fig. 5.
Fig. 7 is a view showing a clothing for infant provided with a living body measuring sensor according to another embodiment of the present invention.
Fig. 8A is an enlarged view of a conductive fiber constituting the living body measuring sensor shown in Fig. 7.
Fig. 8B is an enlarged sectional view of the conductive fiber constituting the living body measuring sensor shown in Fig. 7.
Fig. 9 shows a clothing for infant provided with a living body measuring sensor according to still another embodiment of the present invention.
Fig. 10 shows a clothing for infant provided with a living body measuring sensor according to still another embodiment of the present invention.
Fig. 11 shows a clothing for infant provided with a living body measuring sensor according to still another embodiment of the present invention.
Fig. 12 shows a clothing for infant provided with a living body measuring sensor according to still another embodiment of the present invention.
Fig. 13 shows a sheet of a baby carriage as an example of a sheet for infant provided with a living body measuring sensor according to still another embodiment of the present invention.
Fig. 14 shows a sheet of a baby carriage as an example of a sheet for infant provided with a living body measuring sensor according to still another embodiment of the present invention.
Fig. 15 is a schematic block diagram of a conventional electrocardiograph.
Fig. 16 is a graph showing an electrocardiographic waveform outputted from the conventional electrocardiograph.

### BEST MODE FOR CARRYING OUT THE INVENTION

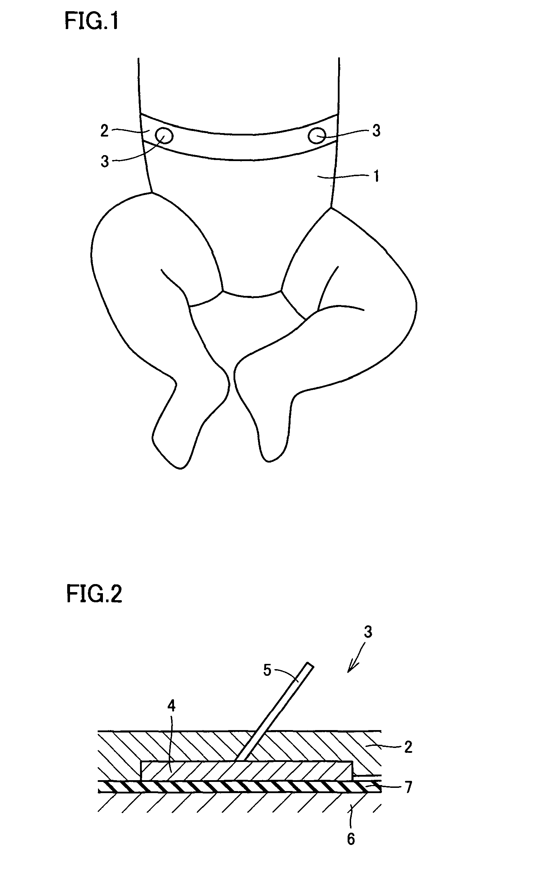

Fig. 1 shows a clothing for infant provided with a living body measuring sensor according to an embodiment of the present invention. Fig. 2 shows the living body measuring sensor incorporated into the clothing for infant of Fig. 1.

In Fig. 1, a clothing for infant 1 such as a diaper cover is provided with a band portion 2 at a position of a waist, and two right and left living body measuring sensors 3 are embedded into the band portion 2. The living boy measuring sensor 3 includes a silver electrode 4 as an example of a metal electrode as shown in Fig. 2. The silver electrode 4 is formed in a thin disk shape or rectangular shape, and a lead wire 5 is connected to one surface of the silver electrode 4. A thin cloth 7 such as silk is provided between the silver electrode 4 and a skin 6 which is a body surface of a measuring subject. The cloth 7 prevents the silver electrode 4 from directly contacting the skin 6. The cloth 7 constitutes a capacitance C between the silver electrode 4 and the skin 6. The band portion 2 is formed from an elastic member such as rubber.

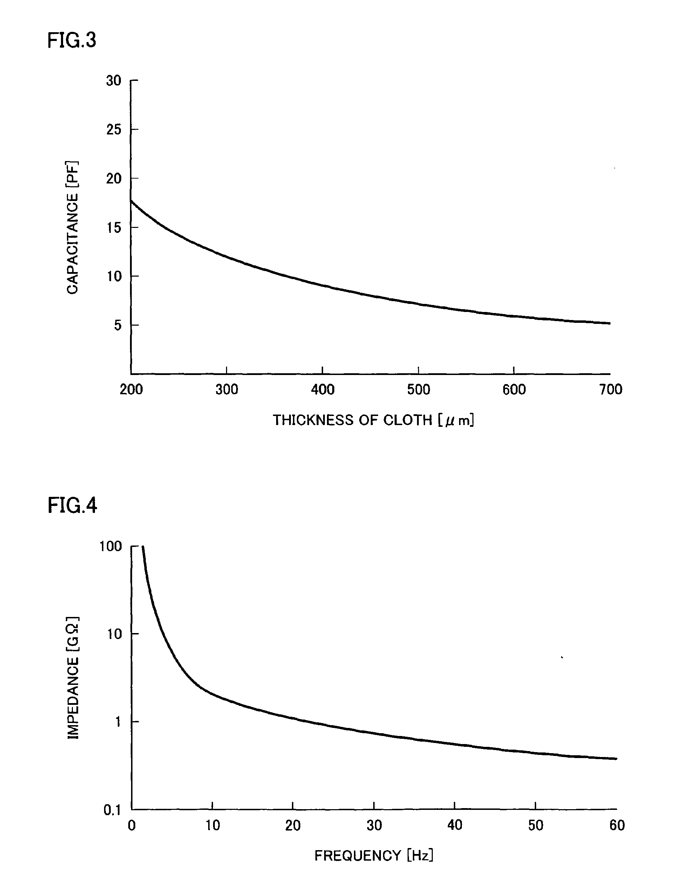

Fig. 3 shows that the capacitance C increases as a thickness of the cloth 7 is getting thinner. Fig. 4 is a graph showing a relationship between a frequency and an impedance.

It is estimated from Fig. 3 that the capacitance between the living body measuring sensor 3 and the skin 6 is approximately 10⁻¹¹ F when a silk having a thickness d of approximately 240 µm is used as the cloth 7. Further, as shown in Fig. 4, an output impedance is lessened as a frequency f of a living-body waveform is increased. An output impedance Z of the living body measuring sensor 3 with the cloth 7 being interposed is estimated to be a high value of approximately 10¹¹ Ω at the frequency of 0.1 Hz.

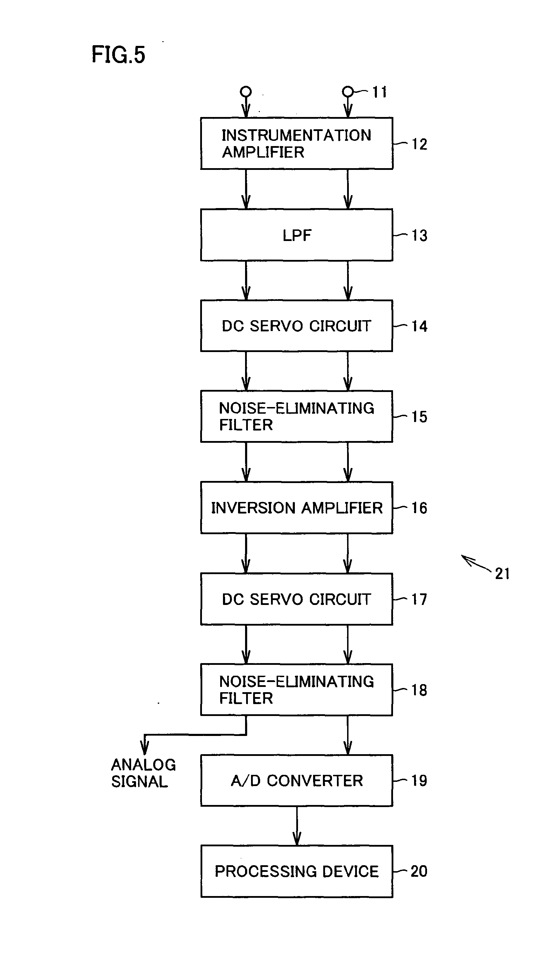

Fig. 5 is a block diagram of a living body measuring device 21 for outputting an electrocardiogram based on a living body electric signal outputted from the living body measuring sensor 3 shown in Fig. 1. As described earlier, the output impedance Z of the living body measuring sensor 3 shows such a high value of 10¹¹ Ω, which results in the generation of a large noise voltage when even a slight amount of noise current is applied to the output. In order to deal with the disadvantage, an impedance converter for outputting the living body electric signal detected and outputted by the living body measuring sensor 3 with a low impedance is necessary.

The living body electric signal of the high impedance detected by the living body measuring sensor 3 is supplied to an instrumentation amplifier 12 via an input terminal 11, and converted into the living body electric signal of the low impedance, and then supplied to an LPF (low-pass filter) 13. In the instrumentation amplifier 12, an input impedance is set to 1000 GΩ, and a gain is set to 62 times as a result of changing a value of an externally added resistance. The LPF 13 extracts a frequency component equal to or below 100 Hz from the living body electric signal and supplies a result of the extraction to a DC servo circuit 14. The DC servo circuit 14 applies the servo so that a variation of a DC component of the living body electric signal is controlled to be zero, which is supplied to a noise eliminating filter 15. The noise eliminating filter 15 is adapted to be switched upon necessity so that the frequency component of 50 Hz or 60Hz can be extracted from the living body electric signal, and supplies the living body electric signal of the extracted frequency component to an inversion amplifier 16.

The inversion amplifier 16 amplifies the living body electric signal, which is inverted by the instrumentation amplifier 12, by 16 times, and inverts the living body electric signal to an initial polarity of the signal. Accordingly, the living body electric signal is amplified by 62×16 ≅ 1000 times. The inverted living body electric signal is supplied to a DC servo circuit 17, which applies the servo in the same manner as the DC servo circuit 14 so that the variation of the DC component of the living body electric signal becomes zero, and supplies it to a noise eliminating filter 18. The noise eliminating filter 18 is adapted to be switched upon necessity so that the frequency component of 50 Hz or 60Hz can be extracted from the living body electric signal in the same manner as the noise eliminating filter 15 in the previous stage. The living body electric signal extracted by the noise eliminating filter 18 is sampled by an A/D converter 19 and converted into a digital signal, and then supplied to a processing device 20 to be subjected to necessary processes. As a result, the electrocardiographic waveform is outputted. As another possible constitution, an analog living body electric signal is outputted from the noise eliminating filter 18 and the electrocardiographic waveform is observed by an oscilloscope.

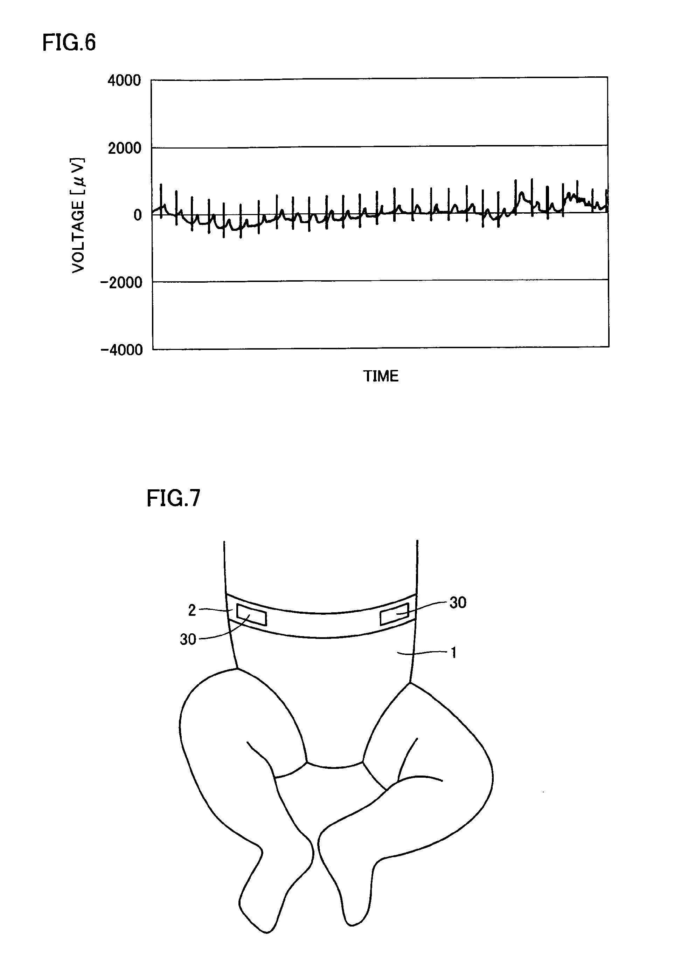

Fig. 6 shows an electrocardiographic waveform outputted from the living body measuring device shown in Fig. 5. As shown in Fig. 6, a relatively stable electrocardiographic waveform could be obtained from the processing device 20.

As described, according to the present embodiment, the living body measuring sensor 3 is incorporated into the band portion 2 of the clothing for infant 1 such as the diaper cover and the silver electrode 4 is brought into a close contact with the skin 6 via the thin cloth 7. Thereby, a risk of an allergic reaction, which could be induced from the direct contact of the metal electrode with the skin 6 for long hours, can be eliminated. Further, such an accident that the skin is removed together with the exchanged electrode can be avoided, and a burden and invasiveness generated from the measurement on the infant can be reduced. As another advantage, such a burden accompanying the measurement as undressing the infant can be lightened to a minimum because the living body measuring sensor 3 is incorporated into the clothing for infant 1 such as the diaper cover.

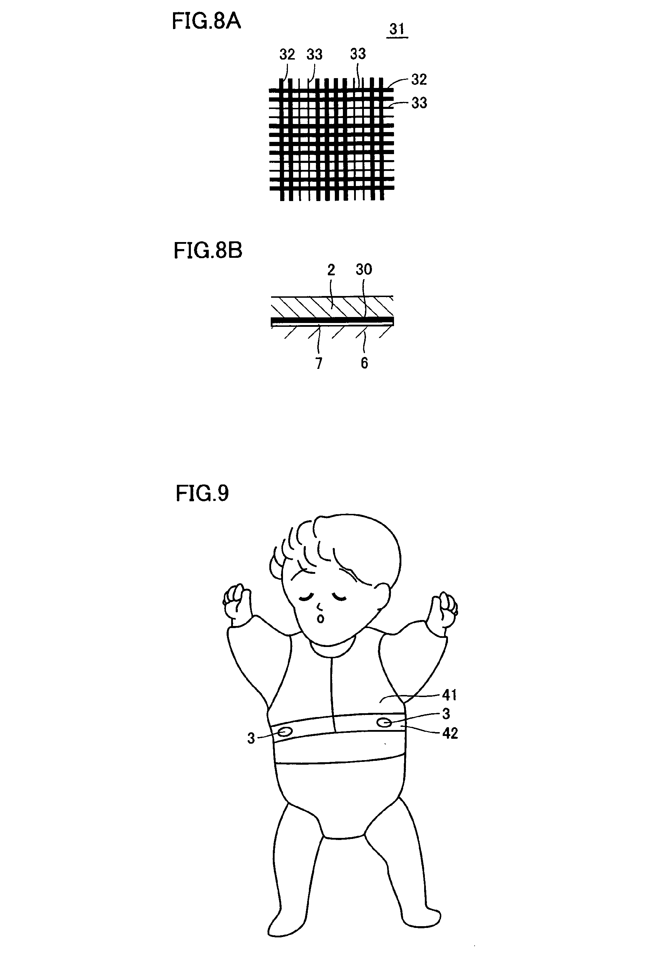

Fig. 7 shows a clothing for infant provided with a living body measuring sensor according to another embodiment of the present invention. Figs. 8A and 8B are enlarged views of a conductive fiber constituting the living body measuring sensor.

In the case of the clothing for infant 1 shown in Fig. 1, the living body measuring sensor 3 formed from the two silver electrodes 4 is embedded into the band portion 2 thereof. According to the embodiment shown in Fig. 7, a living body measuring sensor 30 using a conductive fiber, instead of the silver electrode 4, is incorporated into the band portion 2. More specifically, the living body measuring sensor 30 includes a conductive fabric 31 cut out in a rectangular shape as shown in Fig. 8A. A woven body formed from a conductive yarn 32 and a non-conductive yarn 33 constitutes the conductive fabric 31. As shown in Fig. 8B, a cloth 7 such as silk is incorporated into between the woven body and the skin 6. The cloth 7 constitutes a capacitance between the conductive yarn 32 and skin 6.

The conductive yarn 32 can employ, for example, a metal yarn such as gold, silver or copper, a conductive polymer such as polyaniline or polyacetylene or a conductive fiber such as a silver-plated nylon yarn. The non-conductive yarn 33 can employ a cotton yarn, acryl, nylon, a polyester yarn or the like. Electrically connecting the conductive fabric 31 to the input terminal 11 of the living body measuring device 21 shown in Fig. 5 by means of the lead wire, the electrocardiographic waveform can be outputted from the processing device 20.

Figs. 9 through 12 each shows a clothing for infant provided with a living body measuring sensor according to still another embodiment of the present invention.

According to the embodiment shown in Fig .9, the band portion 2 is provided in a belly portion of an upper wear 41 which is a front-opening underwear for infant, and the two right and left living body measuring sensors 3 are incorporated in the band portion 2 with a predetermined interval provided therebetween.

In the example shown in Fig .10, the two right and left living body measuring sensors 3 are incorporated in vicinity while being vertically and horizontally shifted relative to each other into a breast portion of an upper wear 42 which is an underwear for infant of pullover type. The upper wear 42 is preferably a wear capable of making a close contact with the skin of the infant.

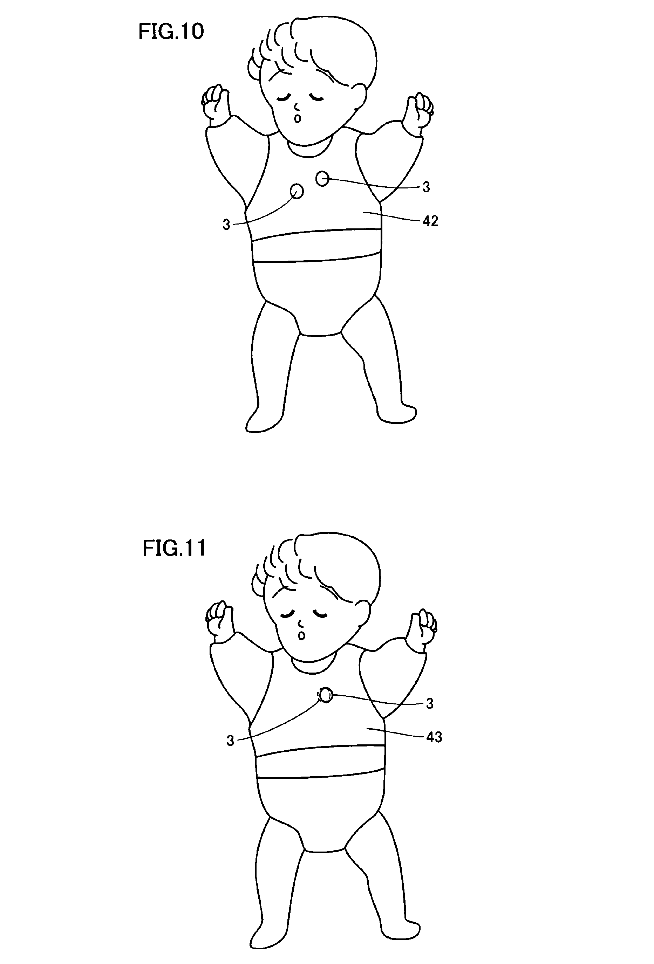

In the example shown in Fig. 11, the living body measuring sensors 3 are incorporated facing each other in a breast portion and a back portion of an upper wear for infant 43 of the pullover type.

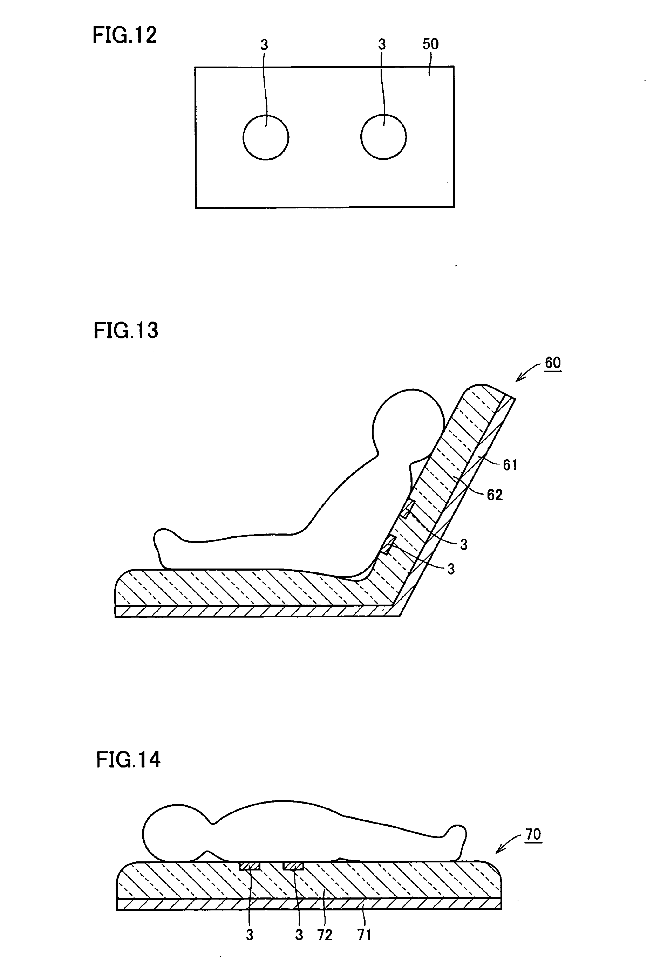

In Fig. 12, the two living body measuring sensors 3 are mounted on a pressure sensitive adhesive sheet 50, and the adhesive sheet is attached to the underwear of the infant. As another possible constitution, a thin cloth is attached to the living body measuring sensors 3, and the adhesive sheet 50 is directly attached to the skin of the infant. In the present invention, the adhesive sheet 50 shown in Fig. 12 is included in the clothing for infant.

Fig. 13 is a sectional view of a sheet for infant provided with a living body measuring sensor according to still another embodiment of the present invention. A sheet 60 of, for example, a baby carriage includes a frame 61 and a cushion 62 provided on the frame 61. The living body measuring sensors 3 are incorporated into positions of the cushion 62 brought into a close contact with the back portion and the waist portion of the infant by a weight of the infant when sat down on the cushion 62.

The living body measuring sensors 3 may be exposed out of a surface of the cushion 62 instead of being incorporated into the cushion 62 and thereby closely contact the back portion and the waist portion of the infant via the underwear. Further, the living body measuring sensors 30 shown in Fig. 7 may be provided in place of the living body measuring sensors 3.

According to the present embodiment, the infant can be monitored by the electrocardiogram continuously measured in order to, for example, assure the growth after birth in the state of sitting the infant on the sheet 60 of the baby carriage or the like.

The sheet 60 is not necessarily provided in the baby carriage. The present invention may be applied to a child sheet, a baby sitting chair of a different type or the like capable of reducing the burden generated from the measurement on the infant.

Fig. 14 is a sectional view of a sheet for infant provided with a living body measuring sensor according to still another embodiment of the present invention. In a sheet 70 of, for example, a bed for infant, a cushion 72 is disposed on a frame 71. The living body measuring sensors 3 are provided at positions of the cushion 72 brought into a close contact with the back portion and the waist portion of the infant by the weight of the infant when laid down on the cushion 72. In the present example, the living body measuring sensors 3 may be exposed out of a surface of the cushion 72 instead of being incorporated into the cushion 72 and thereby closely contact the back portion and the waist portion of the infant via the underwear as in the foregoing example.

Therefore, according to the present embodiment, the infant can be monitored by the electrocardiogram continuously measured in order to, for example, assure the growth after birth in the state of laying down the infant on the sheet 70 of the bed for infant.

The living body measuring sensors 3 may be incorporated into a different object such as a bed mattress and a bed blanket which can be brought into a close contact with the skin of the infant by the weight of the lying infant, or a baby sitting belt making a close contact with the body of the infant when the infant is cradled.

Thus far, the preferred embodiments of the present invention were described referring to the drawings, however, the present invention is not limited to the embodiments shown in the figures. The shown embodiments can be modified and corrected in various manners within the scope identical to the present invention and the scope of its equivalence.

### INDUSTRIAL APPLICABILITY

The present invention, wherein the living body measuring sensor 30 is brought into contact with the skin 6 by means of the capacitance coupling using the cloth 7 between the conductive fabric 31 and the body surface of the measuring subject as the capacitance, the living body electric signal is extracted from the conductive fabric 31, and the output of the living body measuring sensor 30 is supplied to the living body measuring device 25 including the impedance converter having the high input impedance and low output impedance so as to read the voltage waveform, can be utilized in measuring the electrocardiogram in the less invasive manner.

## Claims

1. A clothing for infant provided with a living body measuring sensor for detecting a living body electric signal from a body surface of an infant, comprising a conductive electrode incorporated into said clothing for infant and capacitance-coupled via an insulating member on said body surface of said infant.

2. The clothing for infant provided with a living body measuring sensor as claimed in Claim 1, wherein said conductive electrode is a metal electrode.

3. The clothing for infant provided with a living body measuring sensor as claimed in Claim 1, wherein said conductive electrode is a conductive fiber.

4. The clothing for infant provided with a living body measuring sensor as claimed in any of Claims 1 to 3, wherein said insulating member is a thin cloth.

5. The clothing for infant provided with a living body measuring sensor as claimed in any of Claims 1 to 4, wherein said clothing for infant is a diaper cover, and said conductive electrode and said insulating member are incorporated into a band portion of the diaper cover.

6. The clothing for infant provided with a living body measuring sensor as claimed in any of Claims 1 to 4, wherein said clothing for infant is an upper wear, and said conductive electrode and said insulating member are incorporated into a band portion provided in a lower part of the upper wear.

7. The clothing for infant provided with a living body measuring sensor as claimed in any of Claims 1 to 4, wherein said clothing for infant is an upper wear, and said conductive electrode and said insulating member are incorporated into a breast portion of the upper wear.

8. The clothing for infant provided with a living body measuring sensor as claimed in any of Claims 1 to 4, wherein said clothing for infant is an upper wear, and said conductive electrode and said insulating member are incorporated into a breast portion and a back portion of the upper wear.

9. The clothing for infant provided with a living body measuring sensor as claimed in any of Claims 1 to 4, wherein said clothing for infant is a pressure sensitive adhesive sheet into which said conductive electrode is incorporated.

10. A sheet for infant provided with a living body measuring sensor for detecting a living body electric signal from a body surface of an infant, comprising a conductive electrode incorporated into said sheet for infant and capacitance-coupled via an insulating member on said body surface of said infant.

11. The sheet for infant provided with a living body measuring sensor as claimed in Claim 10, wherein said sheet for infant is a sheet with which said body surface makes a close contact when said infant is sat down.

12. The sheet for infant provided with a living body measuring sensor as claimed in Claim 10, wherein said sheet for infant is a sheet with which said body surface makes a close contact when said infant is laid down.

13. A living body measuring method for extracting a living body electric signal from a body surface of an infant via a conductive electrode, wherein an insulating member is interposed between said body surface of said infant and said conductive electrode to enable capacitance coupling, and a living body electric signal is thereby outputted with a low impedance.

14. The living body measuring method for extracting a living body electric signal as claimed in Claim 13, wherein said insulating member is a clothing worn by said infant.

15. The living body measuring method for extracting a living body electric signal as claimed in Claim 13, wherein said conductive electrode is incorporated into a clothing worn by said infant and outputs said living body electric signal.
